# EUROPEAN PATENT SPECIFICATION

(11) **EP 2 723 882 B1**
(45) Date of publication and mention of the grant of the patent: **01.05.2019**
(21) Application number: 12733001.7
(22) Date of filing: 21.06.2012
(51) Int. Cl.: C12P 35/04

(54) **PROCESS FOR PREPARING 3'-THIOSUBSTITUTED CEPHALOSPORINS EMPLOYING A PENICILLIN G ACYLASE**
VERFAHREN ZUR HERSTELLUNG VON 3 '-THIOSUBSTITUIERTEN CEPHALOSPORINEN UNTER VERWENDUNG EINER PENICILLIN-G-ACYLASE
PROCÉDÉ DE PRÉPARATION DE CÉPHALOSPORINES 3'-THIOSUBSTITUÉES À L'AIDE D'UNE PÉNICILLINE G ACYLASE

(30) Priority: 23.06.2011 EP 11171178
(43) Date of publication of application: 30.04.2014
(73) Proprietor: Centrient Pharmaceuticals Netherlands B.V., 2613 AX Delft (NL)
(72) Inventor: MOODY, Harold, Monro, NL-6100 AA Echt (NL); CUSAN, Claudia, NL-6100 AA Echt (NL); IJPEIJ, Edwin, Gerard, NL-6100 AA Echt (NL)
(74) Representative: de Pauw, Elmar Sebastian David
(86) International application number: PCT/EP2012/061910
(87) International publication number: WO 2012/175585

(56) References cited:
- EP-A1- 1 394 262
- EP-A2- 0 523 585
- WO-A2-2010/072765
- NL-C2- 1 010 506
- US-A- 3 899 394

## Description

### Field of the invention

The present invention relates to a process for the preparation of 3'-thiosubstituted cephalosporins by enzymatic condensation of a 3'-thiosubstituted β-lactam nucleus with a phenylglycine derivative.

### Background of the invention

Enzymatic production of semi-synthetic β-lactam antibiotics by acylation of the parent amino β-lactam moiety with a side chain acid derivative has been widely described (*e.g.* DE 2163792, DE 2621618, EP 339751, EP 473008, EP 1394262, NL 1010506, WO 1992/01061, WO 1993/12250, WO 1996/02663, WO 1996/05318, WO 1996/23796, WO 1997/04086, WO 1998/56946, WO 1999/20786, WO 2005/00367, WO 2006/069984, WO 2008/110527 and US 3,816,253). The enzymes used in the art are in most cases penicillin acylases obtained from *Escherichia coli* and are immobilized on various types of water-insoluble materials (*e.g.* WO 1997/04086).

The above synthetic enzymatic approaches have been described for semi-synthetic penicillins such as amoxicillin and ampicillin and for semi-synthetic cephalosporins such as cefadroxil, cefprozil, cephalexin and cephradine. Typically the latter class of cephalosporins only comprises examples of molecules without substitution at the 3'-position of the β-lactam core.

However, next to the compounds mentioned above, various cephalosporins have been developed with the objective to change, preferably improve, antibacterial properties. Virtually all of these have been, and still are, chemically prepared from fermentatively obtainable cephalosporin C by introduction of alternate groups at the C-3' position and exchange of the aminoadipyl side chain for other side chains.

The introduction of alterations at the C-3' position of the molecule indeed affects the pharmacokinetic and metabolic properties and many successful antibiotics were developed by introduction of thiol-based leaving groups, such as cefamandole, cefatrizine, cefazedone, cefazolin, cefbuperazone, cefmenoxime, cefodizime, cefonicid, cefoperazone, ceforanide, cefotiam, cefpiramide, ceftezole, ceftiofur, ceftriaxone and cefuzonam (Antibiotic and chemotherapy: anti-infective agents and their use in therapy, Ed. R.G. Finch, Elsevier Health Sciences, 2003, Chapter 15 "β-lactam antibiotics: cephalosporins" by D. Greenwood).

However, the pharmacokinetic advantage of introducing thiol-based leaving groups at the 3'-position also turns out to be a major challenge in preparative approaches. Notably this appears to be true for environmentally friendly enzymatic approaches. Although enzymes from *Acetobacter turbidans, Pseudomonas melanogenum* and *Xanthomonas citri* are reportedly used for the preparation of triazole thiomethyl cephalosporins (US 3,899,394), Won et al. (Appl. Biochem. Biotech. 69, 1-9 (1998)) observed that penicillin acylase from *Escherichia coli* CFC-04017 was poisoned by traces of the 2-mercapto-5-methyl-1,3,4-thiadiazole (MMTD) group of cefazolin. Similar observations were made for the 5-mercapto-1-methyltetrazole (MMTZ) group of cefoperazone and cefpiramide and for the 2,5-dihydro-3-mercapto-2-methyl-5,6-dioxo-1,2,4-triazine (TTZ) group of ceftriaxone during approaches to enzymatically hydrolyze an aminoadipic side chain (US 6,642,020). The above observations were attributed to the liberation of small amounts of free thiol, a process that can easily occur under the aqueous circumstances that are normally favorable for enzymatic reactions. And indeed, in contrast with the cephalosporins mentioned earlier, synthetic penicillin acylase catalyzed approaches towards economically attractive 3'-thiosubstituted cephalosporins, such as compounds in which a D-4-hydroxyphenylglycine moiety is 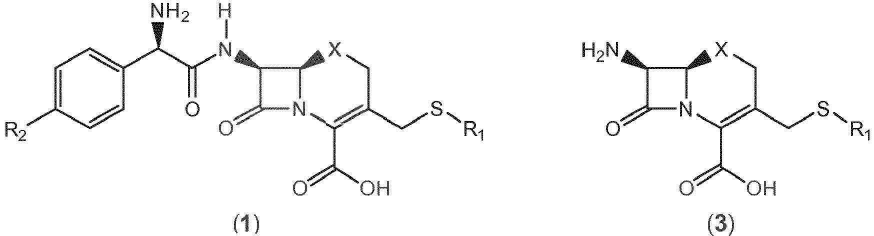 incorporated, have not been reported. The patent document EP 1 394 262 A1 (BIOFERMA MURCIA S.A., 3 March 2004) discloses a process for preparing a compound of general formula (1), characterized in that a compound of general formula (3) is mixed with D-4-hydroxyphenylglycine hydroxyethylester in the presence of a penicillin G acylase at a temperature of from 0°C to 40°C and a pH range of 5.0 to 8.0.

Further, the patent document NL 1 010 506 C2 (DSM N.V., 9 May 2000) discloses a process for preparing a compound of general formula (1), characterized in that a compound of general formula (3) is mixed with an N-substituted D-4-hydroxyphenylglycine amide in the presence of a penicillin G acylase at a temperature of from -5°C to 30°C and a pH range of 5.5 to 9.5. It has been demonstrated that the production of the compound of formula (1) is increased using the N-substituted D-4-hydroxyphenylglycine amide as compared to D-4-hydroxyphenylglycine amide.

Hence, there remains a need to prepare 3'-thiosubstituted cephalosporins such as those of general formula (**1**) by efficient and environmentally friendly enzymatic condensation of a 3'-thiosubstituted β-lactam nucleus of general formula (**3**) with an appropriate side chain.

### Detailed description of the invention

In the context of the present invention, the term "nucleus" is defined as the β-lactam moiety of a 3'-thiosubstituted cephalosporin (*i.e.* a compound of general formula (**3**)) and may for instance be 7-amino-3-(1,2,3-triazol-4(5)-ylthiomethyl)-3-cephem-4-carboxylic acid or 7-amino-3-(1-methyl-1*H*-tetrazol-5-ylthiomethyl)-3-cephem-4-carboxylic acid or the like.

The term "side chain" is defined as the moiety which, in the semi-synthetic β-lactam compound, is attached to the 7-amino position of the nucleus as defined herein, for instance D-4-hydroxyphenylglycine. The term "free side chain" is defined as the acid form of the side chain, for instance D-4-hydroxyphenylglycine. The term "side chain ester" is the ester form of the free side chain whereby the carboxyl group of the free side chain is esterified with an alcohol to give an ester, for instance D-4-hydroxyphenylglycine methyl ester or ethyl ester. The side chain ester may be in the form of the free base or as a salt, for instance as the HCl-salt and the side chain ester may be in a solid form or dissolved in a suitable solvent.

It is an object of the present invention to provide a production process for 3'-thiosubstituted cephalosporins, such as cefatrizine, cefoperazone, cefpiramide or the like or intermediates therefore by enzymatic condensation of a nucleus with a phenylglycine derivative selected from D-4-hydroxyphenylglycine amide or an ester of D-4-hydroxyphenylglycine, or D-phenylglycine amide or an ester of D-phenylglycine.

The nucleus is a compound of general formula (**3**) wherein X represents CH₂ (methylene), O (oxygen) or S (sulfur); preferably X represents S. The radical R₁ preferably is a radical of any of the formula's (**2a**), (**2b**), (**2c**), (**2d**), (**2e**) or (**2f**) 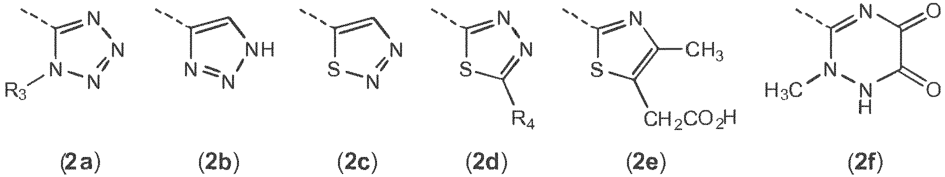 with R₃ is CH₃, CH₂CO2H, CH₂SO₃H, CH₂CH₂OH or CH₂CH₂N(CH₃)₂ and R₄ is hydrogen or CH₃. Most preferably the radical R₁ is (**2a**; R₃ = CH₃) or (**2b**) resulting in nuclei referred to as 7-amino-3-(1-methyl-1*H*-tetrazol-5-ylthiomethyl)-3-cephem-4-carboxylic acid (7-TMCA) and 7-amino-3-(1,2,3-triazol-4(5)-ylthiomethyl)-3-cephem-4-carboxylic acid (7-TACA), respectively.

The phenylglycine derivative preferably is an ester such as D-4-hydroxyphenylglycine ethylester, D-4-hydroxyphenylglycine methylester, D-phenylglycine ethylester or D-phenylglycine methylester. Alternatively the phenylglycine derivative is an amide such as D-4-hydroxyphenylglycine amide, or D-phenylglycine amide. Most preferred the phenylglycine derivative is D-4-hydroxyphenylglycine amide, D-4-hydroxyphenylglycine ethylester or D-4-hydroxyphenylglycine methylester since the 3'-thiosubstituted cephalosporins thus obtained have excellent antibacterial properties (for example cefatrizine) and/or can serve as suitable intermediates in the preparation of cephalosporins with antibacterial properties (for example cefoperazone and cefpiramide).

The most preferred phenylglycine derivative is D-4-hydroxyphenylglycine methylester. Preferably the phenylglycine derivative has the following properties:
- an ee (enantiomeric excess) preferably equal to or greater than 90%, more preferably equal to or greater than 95%, preferably equal to or greater than 96%, preferably equal to or greater than 97%, preferably equal to or greater than 98% and most preferably equal to or greater than 99%; and
- a salt content preferably of 20 mole% or less, more preferably of 10 mole% or less, more preferably of 5 mole% or less, more preferably of 2 mole% or less, most preferably of 1 mole% or less, expressed as moles of salt relative to moles of ester.

It will be evident for the skilled person that an ester in the free base form is provided that can have any value of the ee listed in combination with any value of the salt content listed.

The enzyme used for the enzymatic condensation conveniently is an enzyme suitable for recognizing as substrate amides or esters of α-amino acids such as dihydrophenylglycine, 4-hydroxyphenylglycine and phenylglycine namely penicillin G acylases. In a preferred embodiment it was found that mutant penicillin G acylases as described in WO 2010/072765 are well-suited for the synthesis of 3'-substituted cephalosporins since relatively low amounts of unwanted thiols are generated. Preferably the enzyme is immobilized in order to facilitate separation from the reaction medium and recovery for repeated use. Immobilization can be carried out using a multitude of carrier materials such as silica or gelatin-based carriers.

In a first aspect of the invention there is provided a process for the preparation of a compound of general formula (**1**) 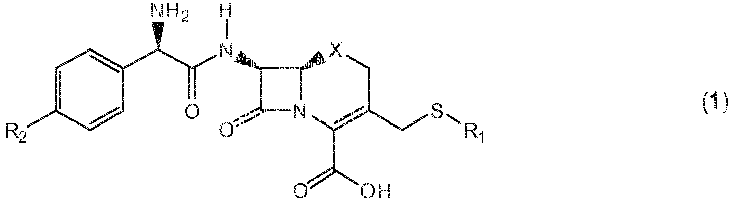 wherein R₁ is a radical of formula (**2a**), (**2b**), (**2c**), (**2d**), (**2e**) or (**2f**) with R₃ is CH₃, CH₂CO₂H, CH₂SO₃H, CH₂CH₂OH or CH₂CH₂N(CH₃)₂ and R₄ is hydrogen or CH₃ 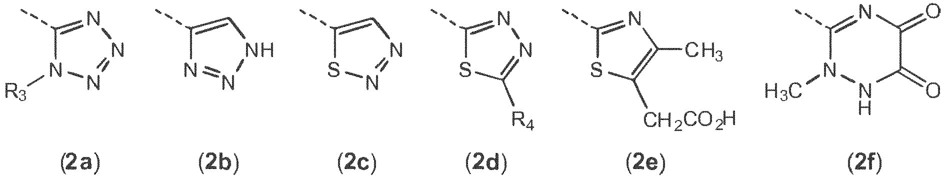 and wherein R₂ is H or OH and X is CH₂, O or S, characterized in that a compound of general formula (3) or a salt thereof wherein R₁ and X are as defined above 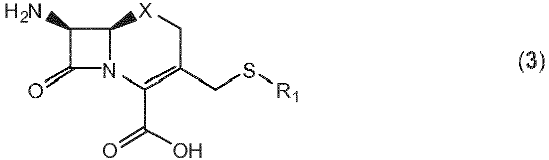 is mixed with D-4-hydroxyphenylglycine amide or an ester of D-4-hydroxyphenylglycine or D-phenylglycine amide or an ester of D-phenylglycine in the presence of a penicillin G acylase at a temperature of from -5°C to 20°C and a pH range of 8.4 to 9.1.

The reaction may be carried out at a wide temperature range, i.e. -5°C to 20°C as the balance between reaction speed, degradation rate and optimal conversion is best tuned within this range. The most optimal temperature range, where high conversions are obtained in combination with low product degradation, was found to be from 0°C to 10°C.

The pH at which the reaction is carried out is from 8.4 to 9.1. The most optimal pH range, where high conversions are obtained in combination with low product degradation, was found to be from 8.5 to 9.0. It was found that it is advantageous when the pH range is from 8.8 to 9.2 in the first 10 to 100 min of the reaction where subsequently the pH is maintained in the range of 8.5 to 8.8. This approach facilitates rapid dissolution of starting material on the one hand while limiting the formation of side products, such as free side chain and thiols on the other hand. It was found that highest conversions are obtained when the starting nucleus is dissolved in the reaction mixture.

It was found that the combination of a reaction temperature range of 0°C to 5°C and a pH range of 8.4 to 9.1 is the most preferred for obtaining the highest yields and lowest side reactions such as formation thiols. The fact that favorable results are obtained in this pH range is unexpected as β-lactams are well known for their instability at basic pH ranges, a fact which is confirmed in EP 1394262 where a pH range of 5.0 - 8.0 is advocated. A still more preferred temperature range is 2°C to 4°C combined with a pH range of 8.6 to 8.8.

The pH-values can be maintained by addition of base during the course of the enzymatic reaction. Suitable bases are ammonia, aqueous potassium hydroxide and aqueous sodium hydroxide. It was found that aqueous sodium hydroxide gives superior results compared to ammonia in terms of co-formation of undesired thiols. In contrast to ammonia, alkaline earth hydroxides such as aqueous lithium hydroxide, potassium hydroxide or sodium hydroxide surprisingly reduced the co-formation of undesired thiols to almost negligible. Preferably the concentration of alkaline earth hydroxide is from 1M to 10M. The skilled person will appreciate that the lower end of this range may be sub-optimal in terms of volume whereas the higher end poses the risk of formation of so-called 'hot spots' that can be detrimental to enzyme and or β-lactam. Thus, a more preferable concentration range of alkaline earth hydroxide is from 2M to 8M, most preferably from 3M to 6M.

Addition of the side chain amide or ester D-4-hydroxyphenylglycine amide or an ester of D-4-hydroxyphenylglycine or D-phenylglycine amide or an ester of D-phenylglycine, can be performed by addition of said side chain amide or ester as a solid or dissolved. When dissolved this is preferably in water wherein the resultant solution is brought at low pH with an acid such as, for example, hydrochloric acid or sulfuric acid. In a preferred embodiment a side chain ester is dissolved and the resulting solution is added to the reaction mixture during a certain time interval. Optionally the side chain ester is added as described in WO 2008/110527 and WO 2008/110529. Said time interval may be from 10 to 300 min, preferably from 30 to 200 min, most preferably from 60 to 180 min.

When the enzymatic coupling reaction has reached the desired degree of conversion, the 3'-thiosubstituted cephalosporin of general formula (1) is recovered using known methods, usually following lowering the pH to a value between 1.5 and 6.5. For instance, a reactor that is equipped with a sieve in the bottom compartment and an outlet at the bottom may be used. The contents of the reactor may then be discharged through the sieve, preferably using upwards stirring. The resulting 3'-thiosubstituted cephalosporin suspension, free of immobilized enzyme, may then be filtered or centrifuged. Due to the low amount of free side chain present after the enzymatic coupling reaction, crystallization of the final 3'-thiosubstituted cephalosporin may be carried out at high concentrations of the 3'-thiosubstituted cephalosporin which results in high yields.

In one exemplifying non-limiting embodiment, where the starting nucleus is (**3**) with R₁ is (**2a**; R₃ = CH₃), R₂ is OH and X is S, the isolation of the corresponding product (**1**) includes removal of immobilized enzyme by filtration, precipitation of the product by lowering the pH with aqueous sulfuric acid, preferably to a value of 2.5 to 6, removal of about 80% water by filtration, dilution of the resulting suspension with methanol and filtration to dryness. Optionally, a further washing step with methanol may be applied. In this way, product (**1**) can be isolated as a stable, manageable solid in 94% yield, and with high quality. Surprisingly, it was found that when methanol is not added during the filtration, the product becomes a sticky gum or clay. The above is applicable to various organic solvents. Preferred solvents are alcohols and ketones such as acetone, ethanol or methanol. In principle the organic solvent can be added to the slurry before filtration, but in this case the impurities present in the mixture (such as D-4-hydroxyphenylglycine methylester, D-4-hydroxyphenylglycine and/or 7-TMCA) lose solubility and may contaminate the final product. Consequently, it is preferred to add the alcohol or ketone after 50-90% of the liquid has been removed from the slurry by filtration. The product of the reaction (**1**) where R₁ is (**2a**; R₃ = CH₃), R₂ is OH and X is S is isolated in a crystalline form which is novel as a result of the hitherto unprecedented enzymatic reaction conditions and downstream processing steps. Advantageously, this crystalline form is highly stable and pure thereby making it an excellent starting material for the high yield, high purity production of both cefoperazone and cefpiramide. The XRD spectrum of the crystalline form shows major peaks at 20 values of 13.9±0.3, 15.6±0.3, 19.1 ±0.3, 19.9±0.3, 22.4±0.3, 23.2±0.3, 24.8±0.3, 28.1±0.3, 29.0±0.3, 32.2±0.3, 33.9±0.3, 38.6±0.3 and 48.8±0.3. Preferably the intensity of any of said major peaks is more than 10% of the intensity of the most intense of said major peaks.

A similar approach is applicable to starting compounds (**3**) wherein R₁ is (**2b**), (**2c**), (**2d**), (**2e**) or (**2f**). When R₁ is (**2b**), R₂ is OH and X is S, the above procedure results in the antibiotic cefatrizine and the product so obtained or a pharmaceutically acceptable salt thereof can be used for the manufacturing of a medicament.

Optionally, and this may be useful in case the 3'-thiosubstituted cephalosporin obtained after enzymatic coupling is derivatized in a subsequent reaction such as outlined below, the 3'-thiosubstituted cephalosporin is not isolated and/or not crystallized.

In yet another embodiment, the product (**1**) obtained by the process of the first aspect described above is further reacted to give a desired pharmaceutical product of general formula (**4**). 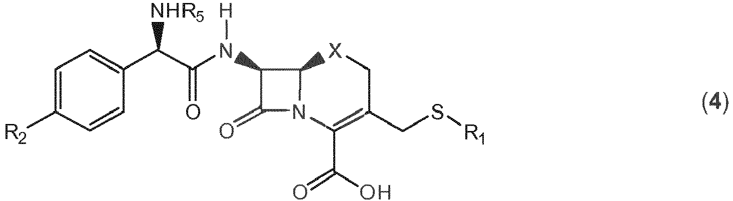 wherein R₁, R₂ and X are as described above and R₅ is a radical chosen from the list consisting of 4-ethyl-2,3-dioxo-1-piperazinecarbonyl, 4-hydroxy-6-methyl-nicotinyl (or the corresponding keto-form 6-methyl-4-oxo-1,4-dihydropyridine-3-carbonyl), 1*H-*imidazole-4-carbonyl-5-carboxylic acid or derivatives such as amides, ethers and esters thereof. Preferably R₁ is a radical of formula (**2a**) with R₃ is CH₃, R₂ is OH, X is S and R₅ is 4-ethyl-2,3-dioxo-1-piperazinecarbonyl or 4-hydroxy-6-methyl-nicotinyl.

Such further reaction preferably comprises derivatization of the amino group of the side chain, for example by reaction with an acid halide in water/ethyl acetate with potassium carbonate, such as described in DE 2600880. After formation and optional isolation of the resulting cefoperazone the product may be converted into a pharmaceutically acceptable salt, preferably the sodium salt, for example by reaction with an inorganic sodium salt such as sodium hydrogencarbonate.

When, instead of 4-ethyl-2,3-dioxo-1-piperazinecarbonylchloride mentioned above, an activated derivative of 4-hydroxy-6-methyl-nicotinic acid (or the corresponding keto-form 6-methyl-4-oxo-1,4-dihydropyridine-3-carboxylic acid) was used, the reaction sequence results in the antibiotic cefpiramide.

The products obtained by the processes of the first aspect, notably cefatrizine, cefoperazone and cefpiramide, are used for the manufacture of a medicament with antibacterial properties. The medicaments thus obtained have the advantage of being produced in high purity and with low environmental burden as compared to their congeners being synthesized chemically.

### Legend to the Figures

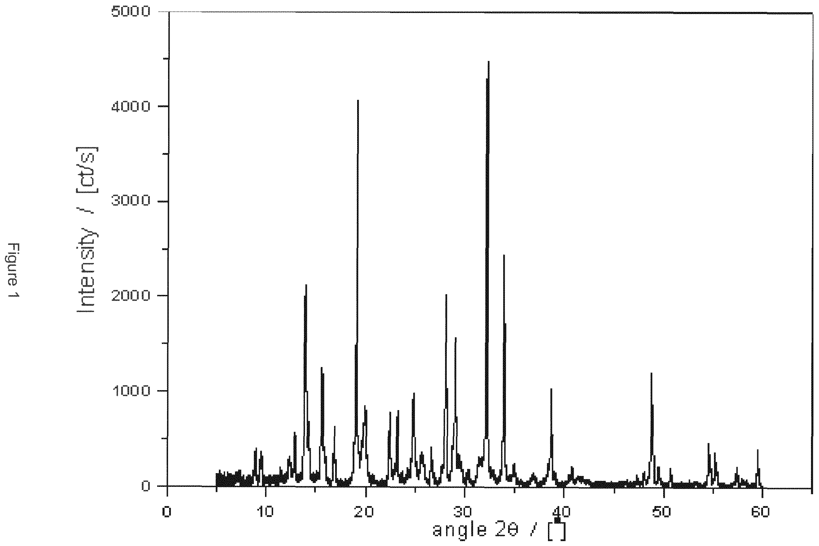

Figure 1 is the X-ray powder diffraction pattern recorded from the compound of formula (**1**) wherein R₁ is a radical of formula (**2a**) with R₃ is CH₃ and wherein R₂ is OH and X is S after storage under ambient conditions for 2.5 h. X-axis: 2-theta value (deg). Y-axis: intensity (cps). The following distinct peaks can be discerned:

| **Angle 2-Theta (deg)** | **d Value (Å)** | **Intensity Count** |
|---|---|---|
| 5,270 | 16,75541 | 95,4 |
| 5,355 | 16,49094 | 134 |
| 6,854 | 12,88602 | 119 |
| 7,180 | 12,30269 | 123 |
| 8,886 | 9,94325 | 259 |
| 9,451 | 9,35066 | 207 |
| 11,418 | 7,74356 | 128 |
| 12,340 | 7,16701 | 218 |
| 12,878 | 6,86899 | 411 |
| 13,905 | 6,36370 | 1479 |
| 14,254 | 6,20847 | 486 |
| 15,153 | 5,84215 | 107 |
| 15,614 | 5,67081 | 856 |
| 16,828 | 5,26434 | 464 |
| 19,059 | 4,65272 | 3026 |
| 19,907 | 4,45640 | 538 |
| 20,686 | 4,29035 | 92,8 |
| 21,545 | 4,12123 | 45,6 |
| 22,404 | 3,96511 | 597 |
| 23,182 | 3,83372 | 626 |
| 23,682 | 3,75394 | 89,6 |
| 24,162 | 3,68048 | 91,2 |
| 24,785 | 3,58938 | 700 |
| 25,618 | 3,47444 | 201 |
| 26,634 | 3,34425 | 299 |
| 27,644 | 3,22426 | 144 |
| 28,053 | 3,17816 | 1583 |
| 28,749 | 3,10279 | 305 |
| 29,010 | 3,07548 | 1186 |
| 29,377 | 3,03792 | 278 |
| 29,620 | 3,01352 | 205 |
| 30,331 | 2,94449 | 127 |
| 31,423 | 2,84457 | 178 |
| 32,145 | 2,78231 | 3879 |
| 32,777 | 2,73013 | 108 |
| 33,868 | 2,64461 | 1968 |
| 34,846 | 2,57261 | 181 |
| 35,627 | 2,51799 | 60,3 |
| 36,794 | 2,44076 | 91,4 |
| 37,939 | 2,36970 | 75,2 |
| 38,367 | 2,34422 | 209 |
| 38,638 | 2,32842 | 904 |
| 38,966 | 2,30957 | 115 |
| 40,479 | 2,22667 | 93,3 |
| 40,778 | 2,21102 | 152 |
| 41,381 | 2,18019 | 84,9 |
| 42,339 | 2,13306 | 58,2 |
| 43,132 | 2,09565 | 44,5 |
| 46,644 | 1,94571 | 32,6 |
| 47,351 | 1,91828 | 108 |
| 48,029 | 1,89277 | 143 |
| 48,797 | 1,86475 | 1064 |
| 49,467 | 1,84106 | 185 |
| 49,804 | 1,82938 | 39,0 |
| 50,689 | 1,79953 | 139 |
| 53,161 | 1,72150 | 43,1 |
| 54,574 | 1,68024 | 389 |
| 55,217 | 1,66218 | 276 |
| 57,351 | 1,60530 | 188 |
| 57,961 | 1,58984 | 71,4 |
| 58,267 | 1,58222 | 44,2 |
| 58,774 | 1,56979 | 34,5 |
| 59,486 | 1,55268 | 366 |

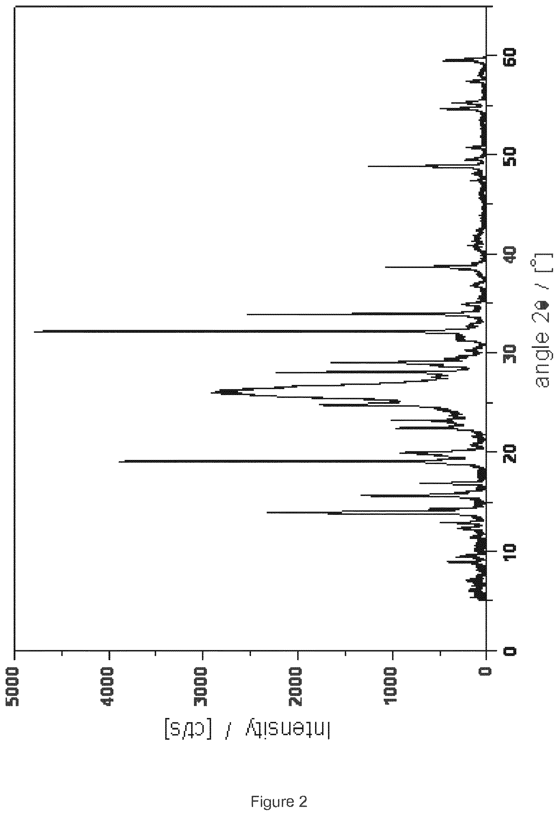

Figure 2 is the X-ray powder diffraction pattern recorded from the compound of formula (**1**) wherein R₁ is a radical of formula (**2a**) with R₃ is CH₃ and wherein R₂ is OH and X is S prior to storage under ambient conditions for 2.5 h. X-axis: 2-theta value (deg). Y-axis: intensity (cps). The discernible peaks are the same as in Figure 1, however there is also a broad maximum at 2θ ∼ 27.

### MATERIALS AND METHODS

### Preparation of immobilized penicillin acylase

The production, isolation and purification of wild type and mutant penicillin G acylases may be carried out as described in WO 1996/005318 and WO 2003/055998. Alternatively, genes encoding mutant penicillin G acylases may be obtained by gene synthesis. Production of the mutant penicillin G acylase was achieved by cloning the genes encoding mutant penicillin G acylases into an appropriate expression vector, transforming a suitable host such as *Escherichia coli* with said vector and culturing the transformed host under conditions suitable for the production of the mutant penicillin G acylases and recovery and purification of the mutants was carried out as described in WO 2010/072765. Penicillin G acylase AA (the *Escherichia coli* wild type penicillin G acylase with mutations B:F24A and B:V148L) and penicillin G acylase mutant 1 (the *Escherichia coli* wild type penicillin G acylase with mutations V11A, A:S3L, A:V192E, B:F24A, B:V148L and B:F460L) as disclosed in Example 1 of WO 2010/072765 were immobilized according to the method disclosed in EP 839192 and EP 222462.

### Analytical HPLC

The reactions were followed by quantitative HPLC analysis. The retention factors of 7-TMCA, HPGM, HPG, 7-TACA and 5-mercapto-1-methyltetrazole were calculated using standards; the retention factor of D-7-(4-hydroxyphenylacetamido)-3-(1-methyl-1*H*-tetrazol-5-yl)thiomethylcephem-4-carboxylic acid was deducted based on mass balance in the first experiments and subsequently calculated using standard; the retention factor of cefatrizine was deducted on mass balance.

| | | | | |
|---|---|---|---|---|
| Instrument: | HPLC Hewlett Packard 1100, detection at 220 nm | | | |
| Column: | Intersil ODS-3, 5µm 4.6x150mm C/N 5020-01731 | | | |
| Flow: | 1 mL/min, stop time 37 min | | | |
| Method: | Eluens A: KH₂PO₃ (5.44 g) and 6 mL H₃PO₄ 1 M, to 2 L with MilliQ water | | | |
| | Eluens B: MeOH | | | |
| | Eluens C: acetonitrile | | | |

| Gradient: | | | | |
|---|---|---|---|---|
| | Time (min) | Eluens A (%) | Eluens B (%) | Eluens C (%) |
| | 0 | 98.5 | 0.5 | 1 |
| | 2.5 | 96 | 3 | 1 |
| | 11 | 74 | 25 | 1 |
| | 15 | 59 | 40 | 1 |
| | 25 | 39 | 60 | 1 |
| | 28 | 19 | 80 | 1 |
| | 32 | 19 | 80 | 1 |
| | 32.1 | 98.5 | 0.5 | 1 |
| | 37 | 98.5 | 0.5 | 1 |

### Measurement of pH values

The pH values referred to in the present invention were measured as follows.

The measurement is performed using 718 STAT Titrino from Metrohm. The pH electrode is from Metrohm, series number 6.0234.110. It contains 3M KCl. The pH meter calibration is performed at 20°C at pH 4 and pH 7 using standard solutions from Merck, using the calibration program present in the instrument.

### XRD measurements

X-ray powder diffraction experiments were performed using a BRUKER D8 ADVANCE diffractometer using standard sample holder with Si-plate to minimize background signal; scan range 5° < 2θ < 60°, sample rotation 30 rpm, 0.3 s/step, 0.00745°/step, divergence slit set to 0.3°; all measuring conditions are fixed in instrument control file CAMBRIDGE.DQL; in addition to sample measurements, corundum reference sample A13-B73 is measured according to conditions outlined in the "instrument calibration" section of the BRUKER manual.

### EXAMPLES

### Example 1

### Enzymatic preparation of D-7-(4-hydroxyphenylacetamido)-3-(1-methyl-1H-tetrazol-5-yl)thiomethylcephem-4-carboxylic acid

### Example 1a

7-Amino-3-(1-methyl-1*H*-tetrazol-5-ylthiomethyl)-3-cephem-4-carboxylic acid (7-TMCA; 5 g) was added to distilled water (38 g) and cooled to 3°C. The mixture was stirred at 400 rpm and the pH was brought to 9.0 with aqueous NaOH (5M) whereafter the remainder of the reaction was carried out at pH 8.8. After 60-80 min, the suspension was filtered. The filtrate, containing 4.1 g of 7-TMCA, was place back in the reactor and immobilized penicillin G acylase mutant 1 (3.5 g, see Materials and Methods) was added and to the resulting mixture a solution of D-4-hydroxyphenylglycine methylester (HPGM) was dropped at speed of 7 mL/h by a syringe pump (dosing time 2 h). This solution was prepared by dissolving HPGM (4.0 g, 1.7 equiv.) in water (6.4 g) and H₂SO₄ 25% (4.25 g in water). The enzymatic reaction was followed by analytical HPLC (see Materials and Methods) and stopped at the end of HPGM addition, by enzyme filtration. The conversion was 98% (w.r.t. 7-TMCA). The mixture contained 1.1% (w/w) D-4-hydroxyphenylglycine (HPG), 0.1% (w/w) 7-TMCA, 1.0% (w/w) HPGM, 7.9% (w/w) of the title compound and 0.07% (w/w) 5-mercapto-1-methyltetrazole. In the HPLC chromatogram two minor not identified impurities were visible with <1% HPLC area percentage.
At the end of the enzymatic reaction, the enzyme was removed by filtration on glass filter no. 1 to give filtrate 65 g at pH 8.8. Under vigorous stirring at 3°C a 25% aqueous solution of H₂SO₄ (2.70 g) was added in 10 min to give a pH of 5.7. The obtained suspension was stirred for 10 min at 3°C and then filtered under vacuum on filter glass no. 3. When 60% of the volume was removed by filtration (40 g mother liquor recovered), MeOH (14 g) was added to the suspension remaining on the filter and the solvent mixture was completely removed by filtration. The final solid was washed with MeOH (15 g). The title product was recovered with a yield of 90% based on the starting material 7-TMCA. Based on HPLC analysis, the mixture contained 0.08% HPG, 0.63% HPGM, 0.41% 7-TMCA and 98.88% of the title compound (data based on HPLC area percentage). ¹H-NMR (2% DCI in D₂O at 300K, on 700 MHz NMR). The values are given in ppm, using TMS as internal reference: 3.5 - 3.7 (dd, 2H), 4.05 (s, 3H), 4.1 - 4.3 (dd, 2H), 5.1 (d, 1H), 5.25 (s, 1H), 5.7 (d,1H), 7.0 (d, 2H), 7.4 (d ,2H). For XRD see Figure 1.

**Example 1b:** as Example 1a with the following differences: the reaction was titrated using 1M sodium hydroxide and the down stream processing performed without MeOH. The conversion was 93% (w.r.t. 7-TMCA). The mixture contained 0.75% (w/w) HPG, 0.3% (w/w) 7-TMCA, 0.2% (w/w) HPGM, 6.6% (w/w) of the title compound and 0.13% (w/w) 5-mercapto-1-methyltetrazole. In the HPLC chromatogram two minor not identified impurities were visible with <1% HPLC area percentage. At the end of the enzymatic reaction, the enzyme was removed by filtration on glass filter no. 1 to give 81 g of filtrate at pH 8.6. Under vigorous stirring at 3°C a 25% aqueous solution of H₂SO₄ (1.6 g) was added in 10 min to give a pH of 7.0. During this operation, a white solid precipitated and was isolated after which the morphology changed into a brown gum.

**Example 1c:** as Example 1b with the following differences: HPGM added as solid during the reaction and the reaction was performed at pH 8.7.
After 240 min, the conversion was 67% (w.r.t. 7-TMCA). The mixture contained 0.43% (w/w) HPG, 1.6% (w/w) 7-TMCA, 0.12% (w/w) HPGM, 6.1% (w/w) of the title compound and 0.12% (w/w) 5-mercapto-1-methyltetrazole.

**Example 1d:** as Example 1c with the following difference: the reaction was titrated using ammonia 25% in water.
After 240 min, the conversion was 63% (w.r.t. 7-TMCA). The mixture contained 0.34% (w/w) HPG, 3.04% (w/w) 7-TMCA, 0.14% (w/w) HPGM, 6.1% (w/w) of the title compound and 0.23% (w/w) 5-mercapto-1-methyltetrazole.

**Example 1e:** as described in the Example 1a with the following differences: the reaction titrated using ammonia 25% in water at pH 8.8 and the down stream processing was carried out without MeOH.
The conversion was 96% (w.r.t. 7-TMCA). The mixture contained 0.88% (w/w) HPG, 0.26% (w/w) 7-TMCA, 0.16% (w/w) HPGM, 7.14% (w/w) of the title compound and 0.9% (w/w) 5-mercapto-1-methyltetrazole. In the HPLC chromatogram two minor not identified impurities were visible with <1% HPLC area percentage. At the end of the enzymatic reaction, the enzyme was removed by filtration on glass filter no. 1 to give 65 g of filtrate at pH 8.8. Under vigorous stirring at 3°C a 25% aqueous solution of H₂SO₄ (3.52 g) was added in 10 min to give a pH of 4.0. During this operation, a white solid precipitated and was isolated after which the morphology changed into a brown gum.

**Example 1f (comparative):** as Example 1e with the following differences: reaction performed at pH 7.2 and 18°C and filtration applied in the down stream processing at the same pH.
After 3 h the conversion was 81% (w.r.t. 7-TMCA). The mixture contained 1.05% (w/w) HPG, 0.78% (w/w) 7-TMCA, 0.02% (w/w) HPGM, 6.4% (w/w) of the title compound and no 5-mercapto-1-methyltetrazole. In the HPLC chromatogram two minor not identified impurities were visible with <1% HPLC area percentage. At the end of the enzymatic reaction, the enzyme was removed by filtration on glass filter no. 1 to give a solid presenting 95% area percentage of desired product and 5% area percentage of starting material 7-TMCA. The NMR analysis of this sample indicated a purity of the product of 83.7%, with 6.8% of 7-TMCA present.

**Example 1g (comparative):** as Example 1d with the following difference: HPGM was added as solid at the beginning of the enzymatic reaction, that was carried out at 10°C and pH from 8.2 to 7.7.
After 245 min, the conversion was 88% (w.r.t. 7-TMCA). The mixture contained 0.77% (w/w) HPG, 0.66% (w/w) 7-TMCA, 0.76% (w/w) HPGM, 10.77% (w/w) of the title compound and not quantified amount of (w/w) 5-mercapto-1-methyltetrazole. The S/H ratio was 9.4.

**Example 1h (comparative):** as Example 1g, using immobilized penicillin G acylase AA instead of immobilized penicillin G acylase mutant 1.
After 295 min, the conversion was 72% (w.r.t. 7-TMCA). The mixture contained 0.18% (w/w) HPG, 2.03% (w/w) 7-TMCA, 2.51% (w/w) HPGM, 8.56% (w/w) of the title compound and not quantified amount of (w/w) 5-mercapto-1-methyltetrazole. The S/H ratio was 18.

**Example 1i (comparative):** as Example 1g with the following difference: HPGM added as solution during the reaction.
After 180 min, the conversion was 95% (w.r.t. 7-TMCA). The mixture contained 0.89% (w/w) HPG, 0.35% (w/w) 7-TMCA, 0.16% (w/w) HPGM, 10.43% (w/w) of the title compound and not quantified amount of (w/w) 5-mercapto-1-methyltetrazole. The S/H ratio was 4.1.

**Example 1j:** as Example 1a with the following differences: reaction was titrated using NaOH 10 M and product was isolated from pH 2.72.
The conversion was 98% (w.r.t. 7-TMCA). The mixture contained 1.12% (w/w) HPG, 0.12% (w/w) 7-TMCA, 1.18% (w/w) HPGM, 8.9% (w/w) of the title compound and 0.04% (w/w) 5-mercapto-1-methyltetrazole. In the HPLC chromatogram two minor not identified impurities were visible with <1% HPLC area percentage. At the end of the enzymatic reaction, the enzyme was removed by filtration on glass filter no.1 to give 59 g of filtrate at pH 8.8. Under vigorous stirring at 3°C a 25% aqueous solution of H₂SO₄ was added in 5 min to give a pH of 2.72. The resulting suspension was diluted with same volume of MeOH and then the product was recovered by filtration. Based on HPLC analysis, the mixture contained 3.8% HPG, 2.9% HPGM, 0.51% 7-TMCA and 92.7% of the title compound (data based on HPLC area percentage).

**Example 1k:** as Example 1j, adding acetone during the down stream process instead of MeOH.
At the end of the enzymatic reaction, the enzyme was removed by filtration on glass filter no. 1 to give 59 g of filtrate at pH 8.8. Under vigorous stirring at 3°C a 25% aqueous solution of H₂SO₄ was added in 5 min to give a pH of 2.72. The resulting suspension was diluted with same volume of acetone and then the product was recovered by filtration. Based on HPLC analysis, the mixture contained 4.3% HPG, 2.3% HPGM, 0.51% 7-TMCA, 0.5% 5-mercapto-1-methyltetrazole and 92.1% of the title compound (data based on HPLC area percentage).

**Example 1l:** as Example 1j with the following differences: reaction performed at pH 8.5 and MeOH added at the beginning of the down stream processing.
The conversion was 97% (w.r.t. 7-TMCA). The mixture contained 1.44% (w/w) HPG, 0.11% (w/w) 7-TMCA, 0.85% (w/w) HPGM, 8.2% (w/w) of the title compound and 0.11% (w/w) 5-mercapto-1-methyltetrazole. In the HPLC chromatogram two minor not identified impurities were visible with <1% HPLC area percentage. At the end of the enzymatic reaction, the enzyme was removed by filtration on glass filter no.1 to give 63 g of filtrate at pH 8.5. Under vigorous stirring at 3°C, the same amount of MeOH was added, followed by 25% aqueous solution of H₂SO₄ to give a pH of 2.78. The mixture recovered by filtration contained 0.2% HPG, 1.5% HPGM, 0.3% 7-TMCA and 98.5% of the title compound (data based on HPLC area percentage).

**Example 1m:** as Example 1l with the following differences: during the down stream processing the compound was precipitated at pH 4.8 and acetone was used during the filtration instead of MeOH.

The conversion was 94% (w.r.t. 7-TMCA). The mixture contained 0.64% (w/w) HPG, 0.36% (w/w) 7-TMCA, 0.64% (w/w) HPGM, 8.37% (w/w) of the title compound and 0.03% (w/w) 5-mercapto-1-methyltetrazole. In the HPLC chromatogram two minor not identified impurities were visible with <1% HPLC area percentage. At the end of the enzymatic reaction, the enzyme was removed by filtration on glass filter no.1 to give 59 g of filtrate at pH 8.6. Under vigorous stirring at 3°C, 5 g acetone was added and the pH dropped to 4.8 by dropwise addition of 2.3 g H₂SO₄ 25% in water at 3°C. The mixture was diluted with 5 g additional acetone and then filtered. By HPLC, the solid on the filter showed 93.5% ABC, 2.8 %HPG, 2.5% HPGM and 1.6% 7-TMCA area percentage. In the mother liquor about 50% of the product was detected. Therefore the solid was mixed with the mother liquor and diluted with 100 g MeOH. After filtration, 73% of the product was recovered by filtration. Based on HPLC analysis, the mixture contained 0.2% HPG, 1.5% HPGM, 0.3% 7-TMCA and 98.5% of the title compound (data based on HPLC area percentage).

### Example 2 (comparative) Degradation of 7-TMCA

During the enzymatic condensation experiments, 7-TMCA underwent chemical degradation, that occurred also in the absence of enzyme, it was faster at basic pH and at high concentration of 7-TMCA. The degradation product was the major side product formed during the enzymatic reaction and was identified by mass analysis to be 5-mercapto-1-methyltetrazole. The chemical degradation occurred using aqueous ammonia (25%) for the titration but was neglectable when aqueous NaOH (1 or 5 or 10M) was applied. Specifically, the amount of 5-mercapto-1-methyltetrazole was not detectable during the dissolution step using aqueous NaOH 5M and 0.05% (w/w) and at the end of the enzymatic reaction; 0.01% (w/w) and 0.24% (w/w) during the dissolution step and at the end of the enzymatic reaction respectively using aqueous ammonia (25%) under the same reaction conditions, or greater under different reaction conditions.

### Example 3 (comparative) Degradation of HPGM

During the enzymatic condensation of 7-TMCA and HPGM the latter compound was hydrolyzed to HPG. As demonstrated below, the formation of HPG was due to enzymatic and not chemical hydrolysis, under the applied experimental conditions. Two reactions were started in parallel at 2°C. In both, water (40 g) of pH 8.7 was mixed with an HPGM solution (0.5 mL in aqueous H₂SO₄, being comparable with the concentration of HPGM present during the enzymatic reactions). In one of the reactors immobilized penicillin G acylase mutant 1 (5 g) was added and the two reactions were monitored by analytical HPLC. Within 5 min, HPGM was completely converted to HPG by the enzyme, while in the chemical blank no HPG was recorded within 2h and only 9% of HPGM was hydrolyzed in 20h.

### Example 4 (falling outside the scope of the invention) Cefoperazone free acid

D-7-(4-Hydroxyphenylacetamido)-3-(1-methyl-1H-tetrazol-5-yl)thiomethylcephem-4-carboxylic acid (500 mg) was suspended at room temperature in water (10 mL). After addition of potassium carbonate (230 mg, 1.5 equiv.) the suspension became a clear solution. To this mixture, ethyl acetate (5 mL) and ethyl-2,3-dioxo-1-piperazinecarbonylchloride (210 mg, 1 equiv.) were added. After stirring overnight, the reaction was analyzed by analytical HPLC showing 42% of the desired product cefoperazone based on the starting material.

### Example 5 (comparative) Enzymatic preparation of cefatrizine

7-Amino-3-(1,2,3-triazol-4-ylthiomethyl)-3-cephem-4-carboxylic acid (7-TACA, 1.03 g, 3.29 mmol) was added to distilled water (50 g) and cooled to 3°C. The mixture was stirred at 400 rpm and the pH was brought to 8.0 with aqueous NaOH (5M). Immobilized penicillin G acylase mutant 1 (5 g, see Materials and Methods) was added and to the resulting mixture a solution of D-4-hydroxyphenylglycine methylester (HPGM) was dropped at speed of 7 mL/h by a syringe pump (dosing time 2 h). This solution was prepared by dissolving HPGM (0.68 g, 3.77 mmol) in water (5 g) and H₂SO₄ 25% (0.5 g in water). The enzymatic reaction was followed by analytical HPLC for 24h (see Materials and Methods; the compounds were identified by comparison of the HPLC retention times with commercially available standards). At the end of HPGM addition, the mixture contained 0.46% (w/w) D-4-hydroxyphenylglycine (HPG), 0.02% (w/w) HPGM, 1.29% (w/w) 7-TACA and 0.77% (w/w) of cefatrizine (the amount of cefatrizine was calculated using a response factor estimated from the mass balance). After stirring the reaction overnight, the product was hydrolysed and the mixture contained 0.91% (w/w) D-4-hydroxyphenylglycine (HPG), 0.01% (w/w) HPGM and 1.93% (w/w) 7-TACA.

## Claims

1. A process for the preparation of a compound of general formula (**1**) 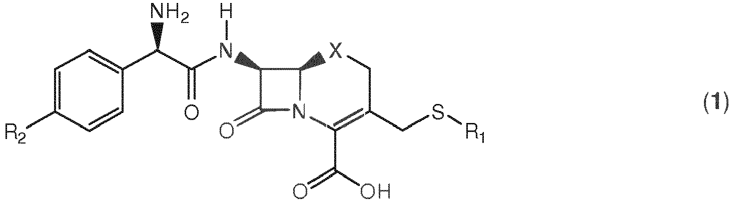 wherein R₁ is a radical of formula (**2a**), (**2b**), (**2c**), (**2d**), (**2e**) or (**2f**) with R₃ is CH₃, CH₂CO₂H, CH₂SO₃H, CH₂CH₂OH or CH₂CH₂N(CH₃)₂ and R₄ is hydrogen or CH₃ 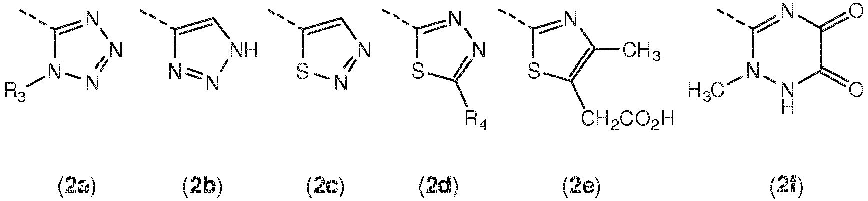 and wherein R₂ is H or OH and X is CH₂, O or S, **characterized in that** a compound of general formula (**3**) or a salt thereof wherein R₁ and X are as defined above 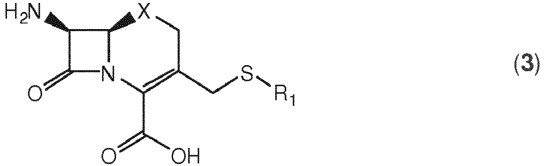 is mixed with D-4-hydroxyphenylglycine amide or an ester of D-4-hydroxyphenylglycine or D-phenylglycine amide or an ester of D-phenylglycine in the presence of a penicillin G acylase at a temperature of from -5°C to 20°C and a pH range of 8.4 to 9.1.

2. Process according to claim 1 wherein said penicillin G acylase is immobilized.

3. Process according to any one of claims 1 to 2 wherein said penicillin G acylase is penicillin G acylase AA or penicillin G acylase mutant 1.

4. Process according to any one of claims 1 to 3 which is carried out between 0ºC and 5ºC.

5. Process according to any one of claims 1 to 4 wherein the pH is maintained by means of adding an aqueous solution of lithium hydroxide, potassium hydroxide, sodium hydroxide or mixtures thereof.

6. Process according to any one of claims 1 to 5 wherein said product of general formula (**1**) is isolated by means of crystallization and filtration or centrifugation.

7. Process according to claim 6 wherein said isolation is preceded by removal of 50-90% of liquid by means of filtration and addition of an organic solvent.

8. Process according to any one of claims 1 to 7 wherein R₁ is a radical of formula (**2a**) with R₃ is CH₃ or a radical of formula (**2b**) and wherein said mixing is with D-4-hydroxyphenylglycine ethyl ester or with D-4-hydroxyphenylglycine methyl ester.

9. Process according to claim 8 wherein R₁ is a radical of formula (**2a**) with R₃ is CH₃ further comprising reacting said compound of formula (1) with a derivative of 4-ethyl-2,3-dioxo-1-piperazinecarboxylic acid or a derivative of 4-hydroxy-6-methyl-nicotinic acid to give cefoperazone or cefpiramide, respectively.

## Patentansprüche

1. Verfahren zur Herstellung einer Verbindung der allgemeinen Formel (**1**) 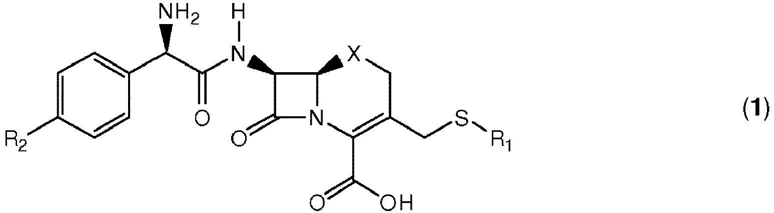 wobei R₁ für einen Rest der Formel (**2a**), (**2b**), (**2c**), (**2d**), (**2e**) oder (**2f**) steht, wobei R₃ für CH₃, CH₂CO₂H, CH₂SO₃H, CH₂CH₂OH oder CH₂CH₂N(CH₃)₂ steht und R₄ für Wasserstoff oder CH₃ steht, 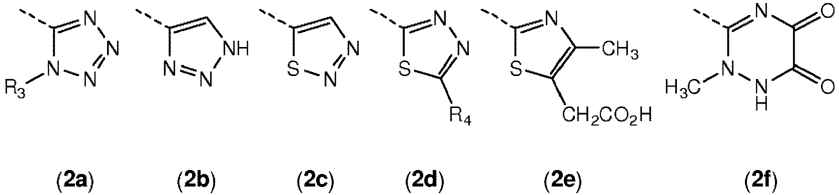 und wobei R₂ für H oder OH steht und X für CH₂, O oder S steht, **dadurch gekennzeichnet, dass** man eine Verbindung der allgemeinen Formel (**3**) oder ein Salz davon, wobei R₁ und X wie oben definiert sind, 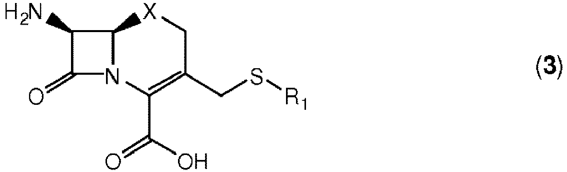 in Gegenwart einer Penicillin-G-Acylase bei einer Temperatur von -5 °C bis 20 °C und einem pH-Bereich von 8,4 bis 9,1 mit D-4-Hydroxyphenylglycinamid oder einem Ester von D-4-Hydroxyphenylglycin oder D-Phenylglycinamid oder einem Ester von D-Phenylglycin mischt.

2. Verfahren nach Anspruch 1, bei dem die Penicillin-G-Acylase immobilisiert ist.

3. Verfahren nach einem der Ansprüche 1 bis 2, bei dem es sich bei der Penicillin-G-Acylase um Penicillin-G-Acylase AA oder Penicillin-G-Acylase Mutante 1 handelt.

4. Verfahren nach einem der Ansprüche 1 bis 3, das zwischen 0 °C und 5 °C durchgeführt wird.

5. Verfahren nach einem der Ansprüche 1 bis 4, bei dem der pH-Wert durch Zugabe einer wässrigen Lösung von Lithiumhydroxid, Kaliumhydroxid, Natriumhydroxid oder Mischungen davon aufrechterhalten wird.

6. Verfahren nach einem der Ansprüche 1 bis 5, bei dem das Produkt der allgemeinen Formel (**1**) durch Kristallisation und Filtration oder Zentrifugation isoliert wird.

7. Verfahren nach Anspruch 6, bei dem vor der Isolierung 50-90 % Flüssigkeit durch Filtration und Zugabe eines organischen Lösungsmittels entfernt werden.

8. Verfahren nach einem der Ansprüche 1 bis 7, bei dem R₁ für einen Rest der Formel (**2a**), wobei R₃ für CH₃ steht, oder einen Rest der Formel (**2b**) steht und wobei mit D-4-Hydroxyphenylglycinethylester oder mit D-4-Hydroxyphenylglycinmethylester gemischt wird.

9. Verfahren nach Anspruch 8, bei dem R₁ für einen Rest der Formel (**2a**) steht, wobei R₃ für CH₃ steht, und ferner die Verbindung der Formel (**1**) mit einem Derivat von 4-Ethyl-2,3-dioxo-1-piperazincarbonsäure oder einem Derivat von 4-Hydroxy-6-methylnicotinsäure zu Cefoperazon bzw. Cefpiramid umgesetzt wird.

## Revendications

1. Procédé de préparation d'un composé de formule générale (1) 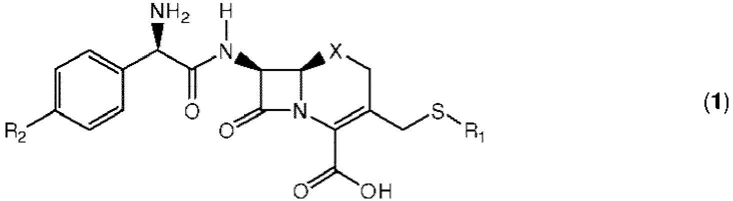 dans lequel R₁ est un radical de formule (2a), (2b), (2c), (2d), (2e) ou (2f) avec R₃ étant CH₃, CH₂CO2H, CH₂SO₃H, CH₂CH₂OH ou CH₂CH₂N(CH₃)₂ et R₄ est hydrogène ou CH₃ 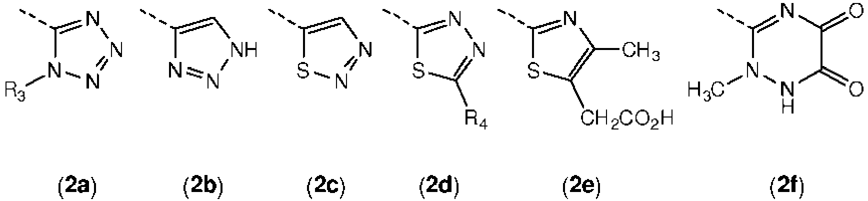 et dans lequel R₂ est H ou OH et X est CH₂, O ou S, **caractérisé en ce qu'**un composé de formule générale (3) ou un sel de celui-ci dans lequel R₁ et X sont tels que définis ci-dessus 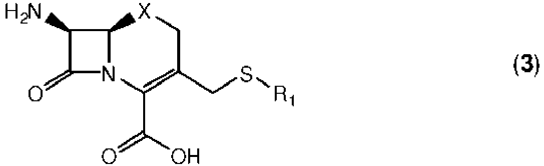 est mélangé avec du D-4-hydroxyphénylglycinamide ou un ester de D-4-hydroxyphénylglycine ou du D-phénylglycinamide ou un ester de D-phénylglycine en présence d'une pénicilline G acylase à une température de -5 °C à 20 °C et une plage de pH de 8,4 à 9,1.

2. Procédé selon la revendication 1, dans lequel ladite pénicilline G acylase est immobilisée.

3. Procédé selon l'une quelconque des revendications 1 à 2, dans lequel ladite pénicilline G acylase est la pénicilline G acylase AA ou la pénicilline G acylase mutante 1.

4. Procédé selon l'une quelconque des revendications 1 à 3, qui est conduit entre 0 °C et 5 °C.

5. Procédé selon l'une quelconque des revendications 1 à 4, dans lequel le pH est maintenu au moyen de l'ajout d'une solution aqueuse d'hydroxyde de lithium, d'hydroxyde de potassium, d'hydroxyde de sodium ou de mélanges de ceux-ci.

6. Procédé selon l'une quelconque des revendications 1 à 5, dans lequel ledit produit de formule générale (1) est isolé au moyen d'une cristallisation et d'une filtration ou centrifugation.

7. Procédé selon la revendication 6, dans lequel ledit isolement est précédé par le retrait de 50 à 90 % du liquide au moyen d'une filtration et l'ajout d'un solvant organique.

8. Procédé selon l'une quelconque des revendications 1 à 7, dans lequel R₁ est un radical de formule (2a) avec R₃ étant CH₃ ou un radical de formule (2b) et dans lequel ledit mélange est effectué avec de l'ester éthylique de D-4-hydroxyphénylglycine ou avec de l'ester méthylique de D-4-hydroxyphénylglycine.

9. Procédé selon la revendication 8, dans lequel R₁ est un radical de formule (2a) dans lequel R₃ est CH₃ comprenant en outre la réaction dudit composé de formule (1) avec un dérivé d'acide 4-éthyl-2,3-dioxo-1-pipérazinecarboxylique ou un dérivé d'acide 4-hydroxy-6-méthyl-nicotinique pour obtenir de la céfopérazone ou du cefpiramide, respectivement.
